# EUROPEAN PATENT APPLICATION

(11) **EP 0 956 863 A1**
(43) Date of publication of application: **17.11.1999**
(21) Application number: 97949255.0
(22) Date of filing: 26.12.1997
(51) Int. Cl.: A61K 38/24

(54) **ORTHODONTIC REMEDY CONTAINING PTH**

(30) Priority: 27.12.1996 JP 35015296
(71) Applicant: Chugai Seiyaku Kabushiki Kaisha, Tokyo 115 (JP)
(72) Inventor: SOMA, Shunichi, Osaka-shi, Osaka 532 (JP); IWAMOTO, Masahiro, Minoo-shi, Osaka 562 (JP); KURISU, Kojiro, Ikoma-shi, Nara 630-01 (JP); HIGUCHI, Yoshinobu, Chugai Seiyaku K.K., Gotenba-shi, Shizuoka 412 (JP)
(74) Representative: VOSSIUS & PARTNER
(86) International application number: JP9704891
(87) International publication number: WO9829130

(57) **Abstract**

Orthodontic remedies containing parathyroid hormone (PTH) or one or more PTH derivatives as the active ingredient(s).

## Description

### TECHNICAL FIELD

This invention relates orthodontic remedies containing parathyroid hormone (PTH) or PTH derivatives as the active ingredient.

### BACKGROUND ART

Parathyroid hormone (PTH) is known as one of the hormones which plays an important role in bone metabolism. There have been reported a number of effects of PTH on bones. In the field of clinical orthodontics, tooth movement is considered to be an accelerated remodeling of a bone due to mechanical stress acting on the tooth. The adaptation of an alveolar bone to such mechanical stress has been shown to constitute an increase in bone resorption in the pressured side of the periodontia and an increase in the bone formation in the strained side of the periodontia. Although attempts have been made to give a clear explanation of these changes taking place in the periodontia under mechanical stress on the basis of the tension hypothesis (Oppenheim, 1911), a detailed cell response mechanism to mechanical stress has not yet been clarified so far (Sandy, Farndale and Meikle, 1993).

It has been considered that acceleration of the bone turnover at a tooth movement is an important factor relating to orthodontic tooth movement. This is because the treatment period can be shortened by accelerating bone turnover. It has been reported that bone resorption can be accelerated in experimental tooth movement by locally or topically administering chemicals such as PGE₁ (Yamasaki, Miura and Suda, 1980; Lee, 1990), PGE₂ (Yamasaki, Miura and Suda, 1980; Chao et al., 1988) and 1α,25-(OH)₂D₃ (Collins and Sinclair, 1988; Takano-Yamamoto, et al., 1992) or systemically administering PGE₁ (Lee, et al., 1988).

It is well known that parathyroid hormone (PTH) is one of the systemic factors required in bone remodeling. Intermittent injection of PTH in vivo brings about an increase in bone mass of ovariectomized (OVX) rats (Hock, et al., 1988; Liu, et al., 1991; Ibbotson, 1992) or normal rats (Hock and Gera, 1992; Dobnig, 1995). It is, therefore, considered that stimulation of bone formation is one of the physiological roles of the pulsating secretion of PTH in vivo. On the contrary, the results of morphological bone measurement indicate that continuous injection of PTH results in the simultaneous acceleration of bone formation and bone resorption but no substantial increase in bone mass either in parathyroidectomized rats (Kitagawa, et al., 1991) and normal dogs (Malluche, et al., 1982). According to the data obtained from various studies in vivo, osteoclast formation (Takahashi, et al., 1988; Kurihara, et al., 1991) and osteoblast proliferation (Somjen, et. al, 1990) are both stimulated by PTH. It is also reported that the administration of PTH exerts systemic effects on the periodontia and, therefore, causes different findings in the alkaline phosphatase reaction in the periodontal ligaments and osteoclast distribution compared with a control group (T.Deguchi, J. Japan Orthodontic Dentistry, Vol. 28, No. 1, 1969, pp. 1-7).

With respect to the role of PTH in bone remodeling relating to orthodontic tooth movement, it has been proved in the above-cited report (Kamata, 1972) that the induction of osteoclasts in the pressured side during an experimental tooth movement is completely inhibited by para-thyroidectomy and then restored by injecting a parathyroid extract. This fact indicates that PTH would play an important role in the osteoclast formation during the experimental tooth movement. However, practical application of PTH in the filed of clinical orthodontics has never been clarified hitherto.

### DISCLOSURE OF THE INVENTION

An object of the present invention is to provide orthodontic remedies which are practically usable and efficacious in the filed of clinical orthodontics.

After conducting extensive studies, the present inventors have found that orthodontic tooth movement is accelerated by administering parathyroid hormone (PTH) or one or more PTH derivatives, thus completing the present invention. Accordingly, the present invention relates to orthodontic remedies containing parathyroid hormone (PTH) or one or more PTH derivatives as the active ingredient(s). The present invention further relates orthodontic remedies containing human PTH (1-84) or one or more derivatives thereof as the active ingredient(s). The present invention further relates to orthodontic remedies containing human PTH (1-34) or one or more derivatives thereof as the active ingredient(s). Furthermore, the present invention relates orthodontic remedies characterized by containing parathyroid hormone (PTH) as the active ingredient. The present invention further relates to orthodontic remedies containing human PTH (1-84) as the active ingredient. The present invention further relates to orthodontic remedies containing human PTH (1-34) thereof as the active ingredient. In addition, the present invention relates to dental compositions containing parathyroid hormone (PTH) or one or more PTH derivatives as the active ingredient(s). Further, the present invention relates to noninvasive PTH preparations characterized by the continuos administration of parathyroid hormone (PTH) or one or more PTH derivatives in an efficacious amount.

The terms "orthodontic dentistry" and "orthodontics" are used herein interchangeably.

The term "orthodontic remedy" as used herein means a drug to be used for correcting abnormalities in teeth or upper and/or lower jaws. The orthodontic remedies of the present invention are employed preferably as drugs for correcting dental irregularities, i.e., remedies for dental irregularity. The term "remedy for dental irregularity" as used herein means a drug to be used for shifting a specific tooth with an abnormality or all teeth into the normal position to thereby normalize a dental arch suffering from some morphological abnormality (i.e., dental irregularity), for example, abnormal interdental distance, tooth malposition (dislocation toward lip (cheek) or tongue).

### BRIEF DESCRIPTION OF THE DRAWINGS

Fig. 1 is a diagram which shows a method for experimentally shifting a tooth by using an elastic band. Fig. 1 (A) shows a method wherein the elastic band is inserted between the first molar (M₁) and the second molar (M₂) by the Wong and Rothblatt method, while (B) and (C) show a method for measuring the distance between M₁ and M₂ with the use of a contact gauge.
Fig. 2 is a graph which shows a dose-dependent effect of PTH (1-84) infusion on teeth separation.
Fig. 3 is a graph which shows a change with the passage of time in the effect of 10 µg/100 g/day of PTH (1-84) infusion into rats on teeth separation.
Fig. 4 is a graph which shows a dose-dependent effect of PTH (1-84) infusion on the appearance of osteoclasts in teeth separation.
Fig. 5 is a graph which shows a change over time in the effect of 10 µg/100 g /day of PTH (1-84) infusion on the appearance of osteoclasts in the pressured side, when an elastic band is inserted between the first molar and the second molar.
Fig. 6 is a diagram which shows a method for orthodontic tooth movement with the use of a closed coil spring. Fig. 6 (A) and (B) show a method wherein an ultra-elastic closed coil spring is ligated between the upper incisive tooth and the right first molar for traction, while (C) shows a method for measuring the shift distance of the tooth with the use of a calipers under a stereoscopic microscope.
Fig. 7 is a graph which shows a dose-dependent effect of hPTH (1-34) infusion on the mesial shift of the first molar.
Figs. 8 and 9 are photographs which show effects of continuous hPTH (1-34) infusion and intermittent hPTH (1-34) injection on the mesial shift of the first molar.
Fig. 10 is a graph which shows changes over time in the effects of continuous hPTH (1-34) infusion and intermittent hPTH (1-34) injection on the mesial shift of the first molar.
Fig. 11 is a graph which shows an effect of local injection of sustained release hPTH (1-34) on the mesial shift of the first molar.
Figs. 12 to 14 are photographs which show effects of local injection of hPTH (1-34) on the shift of the first molar.
Fig. 15 is a graph which shows a change with the passage of time in the effect of local injection of hPTH (1-34) on the shift of the first molar.
Figs. 16 and 17 are photographs which show histological findings of the effects of systemic continuous infusion of hPTH (1-34) and intermittent injection of hPTH (1-34) on the shift of the first molar.
Fig. 18 is a photograph which shows histological findings of the effect of local injection of hPTH (1-34) on the shift of the first molar.

### BEST MODE FOR CARRYING OUT THE INVENTION

The parathyroid hormone (PTH) to be used in the present invention involves natural PTH, recombinant PTHs produced by genetic engineering techniques and chemically synthesized PTHs. Preferable examples thereof include human PTH consisting of 84 amino acid residues (human PTH (1-84)), in particular, recombinant human PTH (1-84) produced by genetic engineering techniques. The term "PTH derivative" involve peptide fragments of the above-mentioned PTHs; peptides constructed by partly substituting the amino acids constituting PTH per se or a peptide fragments thereof by other amino acids; those constructed by partly deleting the amino acids constituting PTH per se or a peptide fragments thereof; and those constructed by adding one or more amino acids to PTH per se or a peptide fragments thereof, each having the same activity. Examples of the peptide fragments of PTH include human PTH (1-34), human PTH (1-64), human PTH (35-84) and bovine PTH (1-34). PTH (1-34) means a peptide fragment of PTH having an amino acid sequence ranging from the amino acid at the N-terminus to the one at the 34-position. As a preferable example of the peptide fragments of PTH, human PTH consisting of 34 amino acid residues (i.e., human PTH (1-34)), in particular, a recombinant human PTH (1-34) constructed by genetic engineering techniques may be cited.

Preferable examples of the amino acid substitution include the substitution of the constituting amino acid at the 8-position by leucine or norleucine, the substitution of the constituting amino acid at the 18-position by leucine or norleucine, and the substitution of the constituting amino acid at the 34-position by tyrosine.

Preferable examples of the parathyroid hormone (PTH) or PTH derivatives to be used in the orthodontic remedies, dental compositions or noninvasive PTH preparations according to the present invention include human PTH (1-84), human PTH (1-34), human PTH (1-38), human PTH (1-37) and human PTH (1-34)-NH₂. Among all, human PTH (1-84) and human PTH (1-34) are still preferable and human PTH (1-34) may be cited as the most desirable one.

It is not always necessary for the parathyroid hormone (PTH) or PTH derivatives to be used in orthodontic remedies, dental compositions or noninvasive PTH preparations according to the present invention have a purity of 100%. Namely, these PTH and PTH derivatives may be substantially pure ones. The term "substantially pure" as used herein means having been purified at least to such an extent as showing a single peak in HPLC, preferably having been identified as being uniform by combining procedures such as SDS-DAGE, capillary electrophoresis, etc. Such PTHs can be proved and identified also by using a method disclosed in JP(Kokai) Hei-6-87897 or methods described in Domestic Announcement No. 4-505259 and J. Biol. Chem., 265, 15854 (1990) which are optionally modified.

The drugs of the present invention may have a dosage form of injections (solutions, freeze-dried preparations, etc.) obtained by a method conventionally employed in producing peptide preparations. Alternatively, they may be in the form of preparations with localized and delayed actions, for example, oral transmucosal preparations produced by packing the drugs in microcapsules or impregnating gel sheets therewith. In the preparation, use can be made of pharmaceutically acceptable auxiliary ingredients. It is also possible to modify the preparations with polyethylene glycol so as to prolong the half-life in blood. Preferable examples of the preparations are noninvasive ones.

Oral transmucosal preparations have been put into practical use with respect to nitroglycerin, nicotin, nifedipine, etc. The advantages of such oral transmucosal preparations as noninvasive peptide or protein preparations reside in that they can be conveniently administered without resort to any specific device, that they suffer from neither digestion in the digestive tract nor the first pass effect in the liver, etc. In the case of hydrophilic substances such as peptides and proteins, however, it is needed to use enhancers to pass through the physicochemical and enzymatic barriers in the oral mucosa, different from the low-molecular weight compounds as cited above. As the enhancers, use can be made of bile acids, dihydrofusidic acids, cyclodextrins, surfactants and chelating agents.

Examples of auxiliary ingredients usable in the preparations of the present invention include bases, stabiilzers, antiseptics, preservatives, emulsifiers, suspending agents, solubilizing agents, solubilizing aids, lubricating agents, corrigents, colorants , perfumes, soothing agents, vehicles, binders, thickening agents and buffer agents. More particularly speaking, it is possible therefor to use, for example, calcium carbonate, lactose, sucrose, sorbitol, mannitol, starch, amylopectin, cellulose derivatives, gelatin, cacao fat, distilled water for injection, aqueous solution of sodium chloride, Ringer's solution, gluose solution, human serum albumin, etc.

To produce the drugs according to the present invention with the use of these auxiliary ingredients, appropriate ones may be selected from among these auxiliary ingredients and employed as stated in, for example, "Iyakuhin Tenkabutsu Ichiran-hyo (List of Pharmaceutical Additives)" (published by Zaidan Hojin Tokyo Iyakuhin Kogyo Kyokai Iji Hoki Iinkai (Committee of Legal Provisions on Medical Affairs, Foundation of Tokyo Pharmaceutical Industry Association) & Osaka Iyakuhin Kogyo Kyokai Iji Hoki Kenkyu Iinkai (Committee of Legal Provisions on Medical Affairs, Osaka Pharmaceutical Industry Association). The amount of each auxiliary ingredient may be appropriately determined within the pharmaceutically acceptable range depending on the dosage form, etc.

The drugs of the present invention may be administered either locally or topically or systemically. When a definite tooth (for example, a front tooth is to be exclusively shifted, local or topical administration is preferable. In particular, continuous local or topical administration is preferable therefor. Preferable examples of methods for the continuous local or topical administration involve topical infusion of PTH with the use of sustained release bases or continuous transmucosal absorption of PTH. Particular examples of the sustained release bases include those usable in submucosal or subperiosteal infusion such as (1) collagen pellets, (2) polylactic acid bases, (3) hydroxyapatite cement, and (4) alginic acid gel. Alternatively, use may be made of patches for transmucosal administration. On the other hand, the advantage of the systemic administration resides in that PTH can be administered without any invasion by using an elaborately planned administration method of this type. Preferable examples of the systemic administration method include subcutaneous administration, intravenous administration, nasal administration and transpulmonal administration. When it is desired to quickly shift all teeth, systemic administration is seemingly superior in convenience to local or topical administration over a broad scope.

The administration period may be determined depending on the cause of the diseases by a clinical dentist based on the period required for shifting the target tooth and fixing the thus shifted alveolar bone. The administration frequency may range from once three months to everyday. It is preferable to administer PTH once a month to 5 times per week, or everyday. Continuous administration is particularly preferable.

The administration dose of PTH according to the present invention may vary depending on the tooth shifting distance, tooth type, the number of the teeth to be shifted, etc. In the case of systemic administration, the dose of PTH ranges from 0.1 µg to about 10 mg, preferably from 10 µg to 1 mg.

### Examples

To further illustrate the present invention in greater detail, the following Examples will be given. However, it is to be understood that the present invention is not restricted thereto.

### Materials

Details (type and manufacturer) of the animals and chemicals employed in these examples were as follows. Male Wister rats (350 to 400 g) were obtained from Oriental Yeast Co., Ltd. (Tokyo). PTH employed in Example 1 was recombinant human PTH (1-84) which had been produced by using a modification of the methods described in Domestic Announcement No. 4-505259 and J. Biol. Chem., 265, 15854 (1990). PTH employed in Example 2 was recombinant human PTH (1-34) manufactured by Peptide Institute Inc., (Mino). Osmotic pumps (Alzet 2ML1) (were purchased from Alza (Palo Alto, Calif. USA). Tween-80 was purchased from Wako Pure Chemical Industries, Ltd. (Tokyo). Elastic bands for orthodontics (Quick-Stik, A-1) were purchased from Unitek (Monrovia, Calif., USA).

### Method of experimental tooth movement

There are two methods for experimental tooth movement, i.e., one wherein an elastic band (made of rubber) is inserted between teeth and another one wherein a closed coil spring for orthodontics is used. The closed coil spring employed herein is Sentalloy closed coil spring 509-21 (made of Ni-Ti, manufactured by Tomy International K.K.) which can give an almost constant traction force of about 30 g within a spring-elongation range of 2 to 3 mm. That is to say, it shows no increase in the traction force in proportion to the spring-elongation, as observed in conventional springs. When the initial elongation of the spring is regulated within the above-mentioned range, therefore, a constant force can be applied regardless of orthodontic tooth movement. When a rubber substance is inserted between molars, an extremely large force is applied immediately after the insertion but scarcely any force is applied after the target tooth is shifted by 0.5 mm. Accordingly, either the method with the insertion of an elastic (rubber) band or another one with the use of a closed coil spring may be used in experimental tooth movement within a short period of time. When a tooth is to be shifted over a long time, however, it is appropriate to use the latter method wherein the first molar is mesially shifted by elongating a closed coil spring. The method with the insertion of an elastic (rubber) band was employed in Example 1, while another method with the use of a closed coil spring was employed in Example 2.

### Example 1

### Experiment 1: Dose-dependent effects of PTH infusion on experimental tooth movement

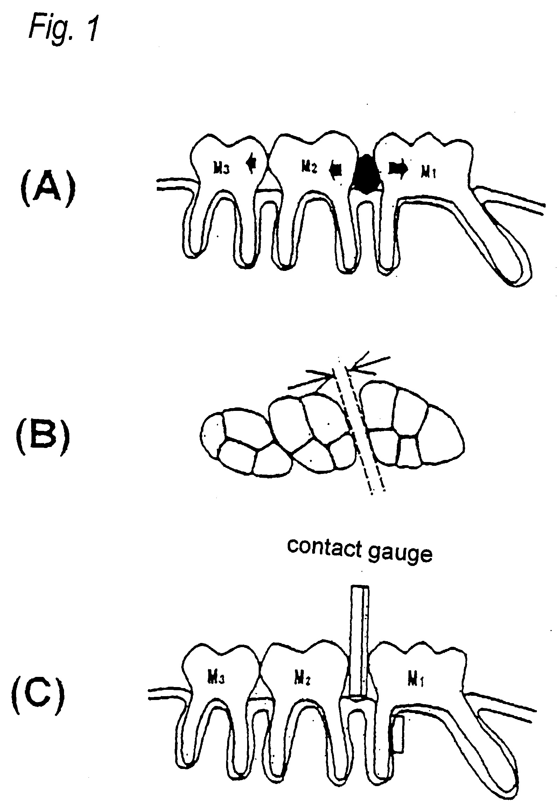

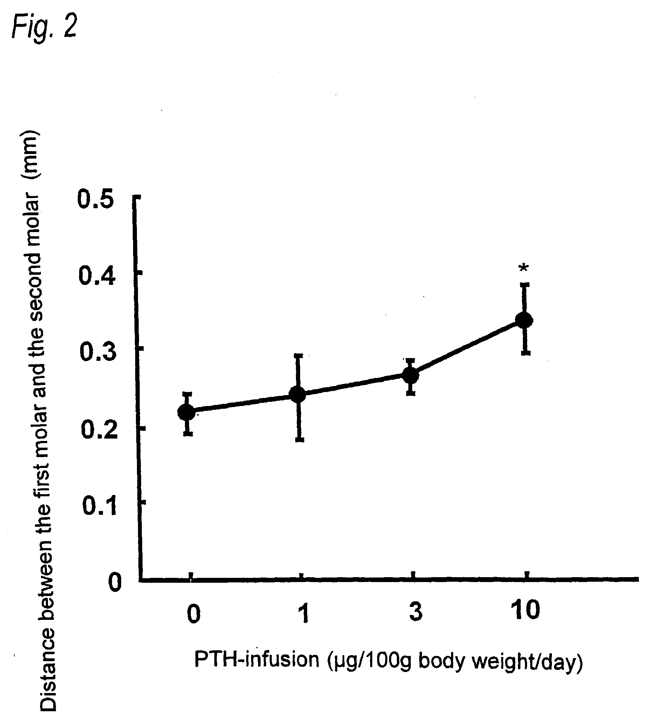

18 rats were divided into 4 groups including a control group having 6 animals and 3 PTH-infusion groups each having 4 animals. PTH was dissolved in a citric acid-buffered saline containing 0.05% of Tween 80 and introduced into osmotic pumps. Then these pumps were implanted in the subcutus posterior region of the neck of the rats under ether anetheia. PTH was continuously infused into the rats in doses of 1, 3 and 10 µg/100 g body weight/day and the rats were fed with a standard pelletized feed (manufactured by Oriental Yeast Co., Ltd.). To the rats of the control group, vehicles were exclusively administered. 48 hours after implanting, a piece of an elastic band (0.8 mm in thickness) was inserted between the right upper first molar and second molar (between M₁ and M₂) of each rat under ether anetheia in accordance with the method of Wong-Rothblatt (1954) as shown in Fig. 1(A). On day 3 of the teeth separation, the rat was sacrificed by ether-inhalation. After cutting out the upper jaw, the distance between the adjacent faces of M₁ and M₂ was measured with the combined use of contact gauges (manufactured by Sun Dental, Osaka) of 50, 100 and 150 µm in thickness (Fig. 1 (B) and (C)). In the case of the control group, a contact gauge of 50 µm could be inserted between M₁ and M₂. Thus, the interdental distance was calculated by subtracting 50 µm from the measured distance of each animal. Fig. 2 shows the results wherein "*" means that a significant difference from the control group (PTH administration: 0 µg/100 g body weight/day) is observed at a significance level of 5%. Fig. 2 indicates that the maximum effect was achieved by infusing PTH in a dose of 10 µg/100 g body weight/day. The upper jaw was fixed in 4% paraformaldehyde, decalcificated in 4% formic acid and then implanted in paraffin. Subsequently, these preparations were cut into continuous mesiodistal sections of 8 µm in thickness and stained with hematoxylin and eosin. Histological examinations were all performed in the pressured side of the M₁ interradicular septa and osteoclasts in the area of 300 x 700 µm² in this region were counted (Fig. 1 (C)). Osteoclasts were counted based on the fact that they were large multinuclear cells having been stained with eosin and located adjacent to the bone surface. The statistic differences between the control group and the test group was evaluated in accordance with Wilcoxon's rank-sum calibration method. Each of the data was expressed in average ± SEM. A P value less than 0.05 was regarded as being statistically significant.

### Experiment 2: Change with the passage of time in effects of PTH infusion on experimental tooth movement

32 rats were divided into 2 test groups each having 16 animals. PTH was infused into these rats in a dose of 10 µg/100 g body weight/day, since it had been revealed by the above Experiment 1 that the maximum effect could be established at this dose. To the rats of the control group, vehicles were exclusively administered. 2 days after implanting osmotic pumps, an elastic band was inserted between the right M₁ and M₂ of each rat. On days 0, 1, 3 and 5 of the teeth separation (i.e., on days 2, 3, 5 and 7 of the PTH infusion), the rats were sacrificed followed by the same procedures as those performed in Experiment 1.

### Results

### 1. Effects of PTH (1-84) infusion on teeth separation due to elastic band:

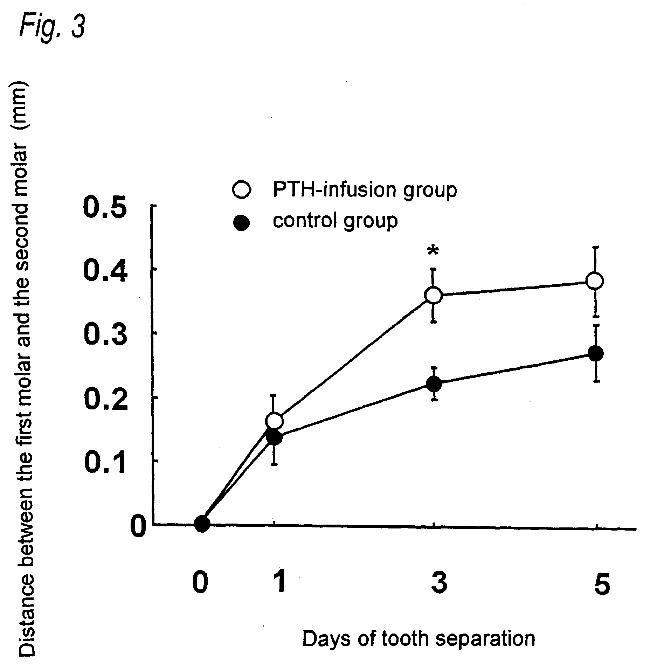

Fig. 2 shows the dose-dependent effect of PTH (1-84) infusion on teeth separation. As reported in a number of papers, teeth separation between M₁ and M₂ arose after inserting the elastic band for 3 days. Compared with the rats of the control group, the rats to which 10 µg/100 g body weight/day of PTH was infused showed a significant increase in the distance between M₁ and M₂. Fig. 3 shows a change with the passage of time in the effect of 10 µg/100 g body weight/day of PTH (1-84) infusion into rats on teeth separation wherein "*" means that a significant difference from the control group on day 3 was observed at a significance level of 5%. On day 1, no significant difference was observed between the rats of the control group and the PTH-treated rats. On day 3, however, the treated rats showed a significant increase in the separation distance. On day 5 of the teeth separation, the separation in the PTH-treated rats seemingly almost reached the limit. On day 5, scarcely any friction was observed between the teeth and the elastic band in the PTH-treated rats and, therefore, the experiment was ceased.

### 2. Effect of continuous PTH (1-84) infusion on the number of osteoclasts in the pressured side of periodontia:

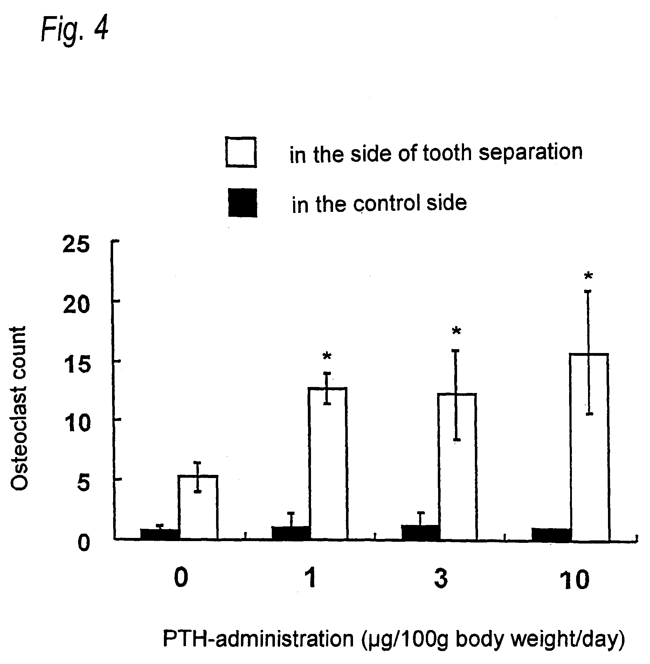

Fig. 4 shows a dose-dependent effect of PTH (1-84) infusion on the appearance of osteoclasts in teeth separation wherein "*" means that a significant difference from the control group (PTH administration: 0 µg/100 g body weight/day) is observed at a significance level of 5%. As having been reported in a number of papers, osteoclasts in the pressured side of the periodontia were increased after inserting the elastic band for 3 days. Different from the results with respect to the teeth separation, the control group showed a significant increase in the osteoclast count compared with all of the 3 test groups.

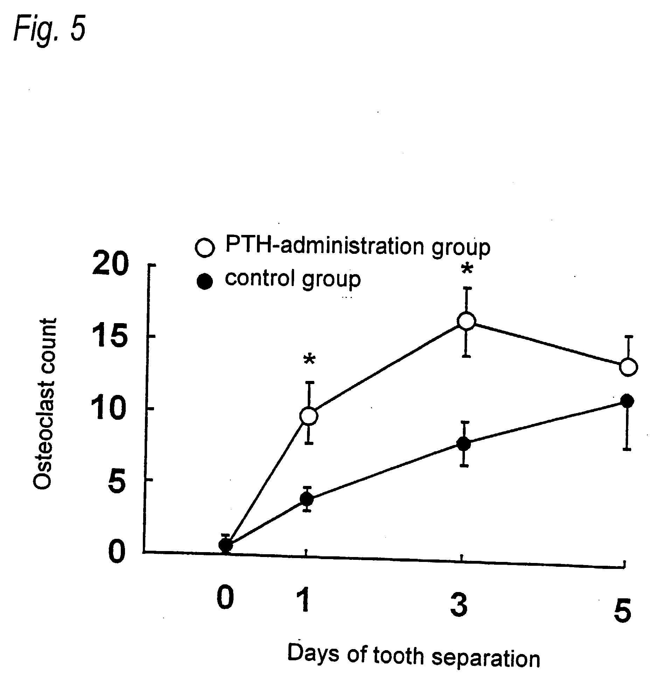

Fig. 5 shows a change with the passage of time in the effect of 10 µg/100 g body weight/day of PTH (1-84) infusion on the appearance of osteoclasts in the pressured side, wherein "*" means that a significant difference from the control group was observed on each day at a significance level of 5%. The osteoclast count showed a significant increase from day 1 of the PTH infusion. On day 5, no significant difference was observed in the osteoclast count between the rats of the control group and the PTH-treated ones.

### 3. Histological change in pressured periodontia of rats with continuous PTH (1-84) infusion:

Histological remodelings of the pressured periodontia in the teeth separation were located exclusively in the mesial periodontium of the mesiobuccal face of M₁. On day 1 of the teeth separation, necrotic tissue was observed both in the vehicle-treated rats (i.e., the rats of the control group) and the rats treated with 10 µg/100 g body weight/day of PTH. On day 3 of the teeth separation, the vehicle-treated rats still showed necrotic tissue in the pressured side. However, the rats treated with 10 µg/100 g body weight/day of PTH showed no necrosis in the same region on day 3 any more. In the rats treated with 1 µg/100 g body weight/day of PTH and those treated with 3 µg/100 g body weight/day of PTH, bone resorption seemingly arose over wider range than in the rats treated with the vehicle. In the rats of these 2 groups, however, necrotic tissue was also observed.

### Example 2

### Experiment 1: Effect of continuous systemic PTH-infusion on experimental tooth movement

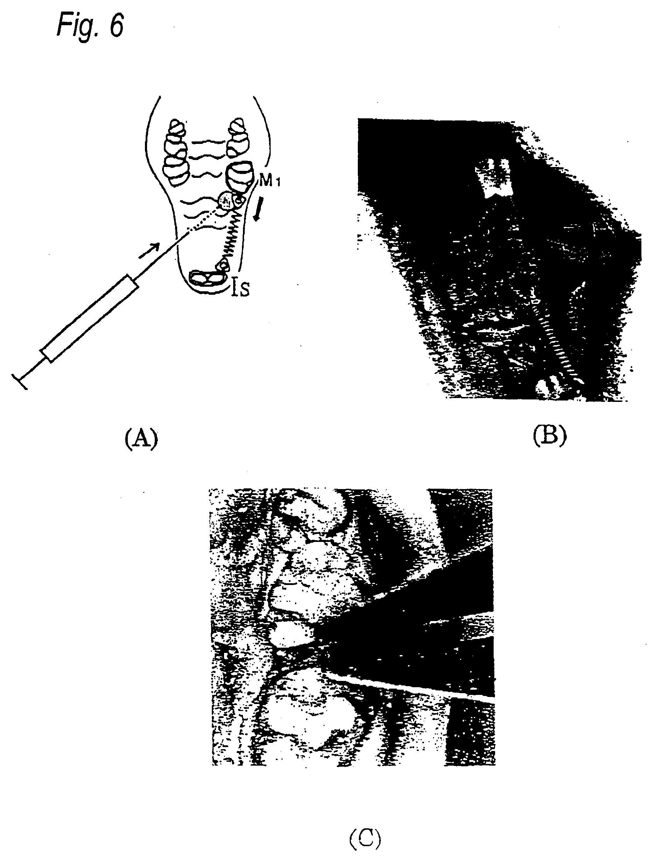

It has been already clarified that the continuous administration of PTH and the intermittent administration thereof differ from each other in their effect on bones. Thus, attempts were made to examine how PTH administered by either method would affect the experimental tooth movement. Use was made of 12 male Wister rats weighing 350 to 400 g. In the continuous PTH-administration group, an osmotic pump (2ML2, manufactured by Alzet, Palo Alto, Calif., USA) was subcutaneously implanted into the dorsal part of each rat followed by the continuous infusion of hPTH (1-34) (manufactured by Peptide Institute Inc., Mino) in a dose of 0.4 µg/100 g body weight/day or 4 µg/100 g body weight/day. As the control groups, use was made of 2 groups including (1) one to which the vehicle was continuously infused, and (2) an intermittent administration group to which 4 µg/100 g body weight/day of PTH was subcutaneously injected into the dorsal part once a day. From the next day of the initiation of the PTH-administration, an ultra-elastic closed coil spring (509-21, manufactured by Tomy International, K.K., Tokyo) was put between the upper incisive tooth and the right first molar M₁ followed by the mesial traction of M₁ for 12 days (Fig. 6-A, B). After the initiation of the shifting, the precise impression of the upper jaw was taken every 3 days by using a silicone impression material (Exafine, manufactured by GC, Tokyo) and the distance between the first molar and the second molar (M₂) was measured on ultra-hard gypsum models (Fig. 6-C). After the completion of shifting over 12 days, arterial blood was collected from the abdominal aorta and various serum parameters were measured. After sacrificing each rat, the upper jaw and the right thickening bone were taken out. The upper jaw was decalcificated and then cut into a paraffin section of 8 µm in thickness involving, in the direction of the major axis, the buccal mesial root and the buccal distal root followed by HE-staining and histological observation. The bone mineral content and bone mineral density of the isolated the femur were measured by dual-x-ray absorptometry (DCS-600, manufactured by Aloka, Tokyo, Japan).

### Experiment 2: Effect of local administration of hPTH (1-34) in sustained release dosage form on experimental tooth movement

To continuously release PTH having been locally administered, 0.1 µg/µl and 1 µg/µl PTH preparations containing 2% of methylcellulose as the base (PTH-MC) were prepared by mixing respectively 0.2 µg/µl and 2 µg/µl PTH solutions in physiological saline with the same amount of 4% methylcellulose. Then these PTH-MC preparations were injected subperiosteally into the palatal mucosa in the mesial palatal side of M₁ in a dose of 1 µl every 2 days (corresponding respectively to 0.0125 µg/100 g/day and 0.125 µg/100 g/day as expressed in Experiment 1) with the use of microsyringes (manufactured by Hamilton) (Fig. 6-A). As the control groups, the following 3 groups were employed: (1) a group to which 1 µl of 2% methylcellulose (MC) alone was injected every 2 days into the same site; (2) one to which 1 µl of a 1 µg/µl solution of PTH in physiological saline (PTH-physiological saline solution) was injected every 2 days into the same site; and (3) one to which 1 µg/µl of PTH-MC was subcutaneously administered every 2 days to the dorsal part. The evaluation of the tooth movement and the histological observation were performed each in the same manner as the one employed in Experiment 1.

### Results

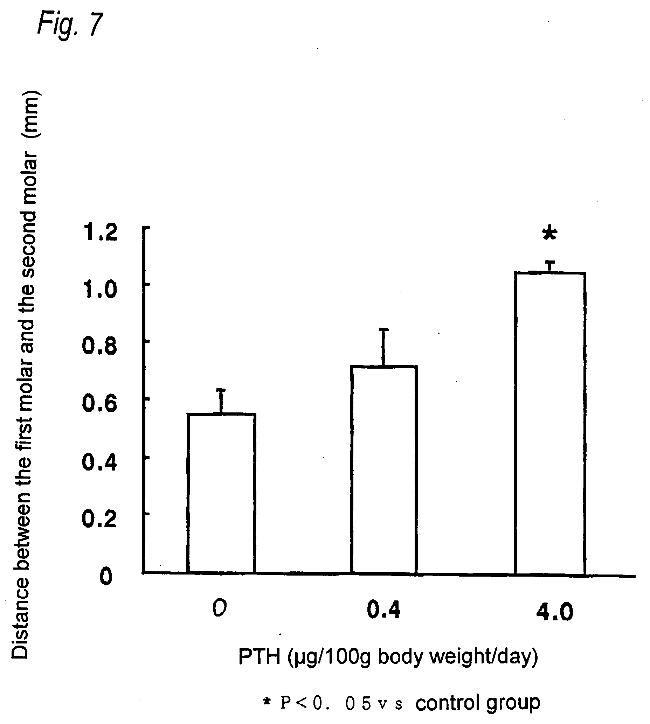

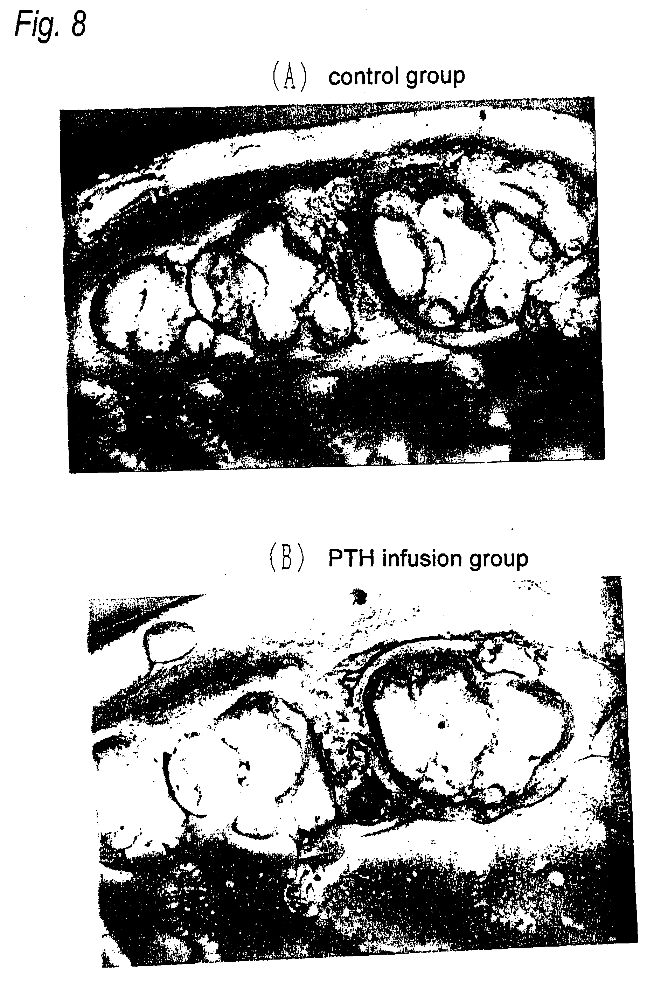

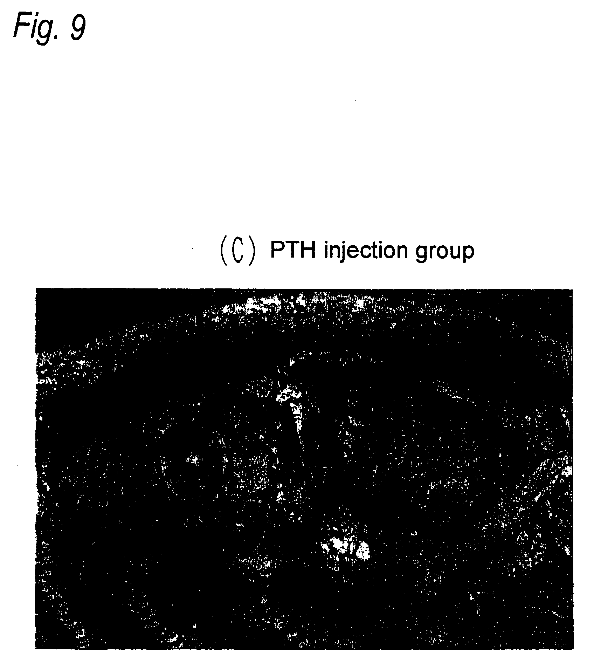

### 1. Effect of continuous systemic PTH-infusion on experimental tooth movement (Figs. 7, 8 and 9).

On day 12 of the tooth movement, the control group showed a mesial shift of M₁ of 0.56 ± 0.04 mm. In the PTH-administration groups, on the other hand, the shift of M₁ was promoted depending on the dose by the continuous infusion of PTH. Namely, the group with the continuous infusion of 4 µg/100 g body weight/day showed a shift of 1.01 ± 0.09 mm, i.e., almost twice as much as that of the control group.

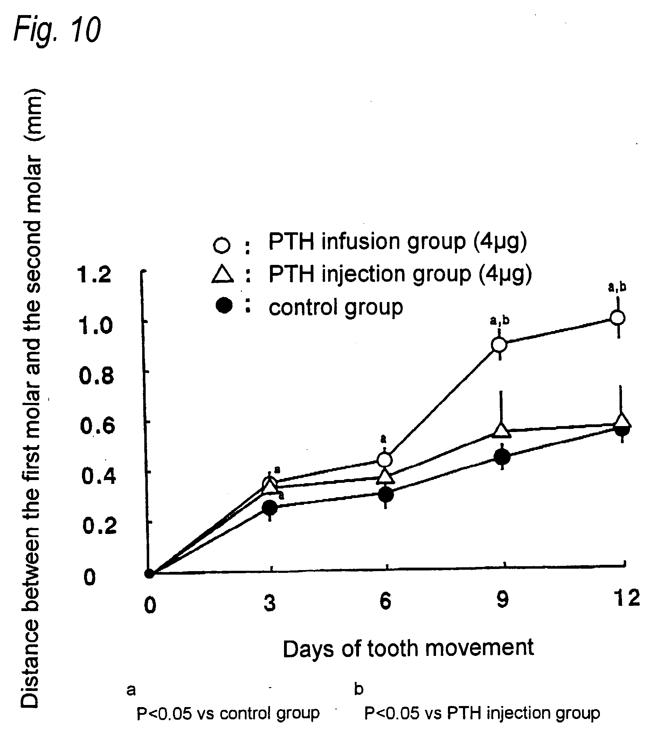

### 2. Change with the passage of time in effect of continuous PTH infusion and intermittent PTH injection on experimental tooth movement (Fig. 10).

On day 3 of the tooth movement, the mesial shift of M₁ was slightly significantly promoted in both of the continuous PTH infusion group and the intermittent PTH injection group. After day 9 of tooth movement, this tooth shift-promoting effect was observed more remarkably in the continuous PTH infusion group. In the intermittent PTH injection group, however, no significant difference from the control group was observed in the M₁ shifting distance after day 6.

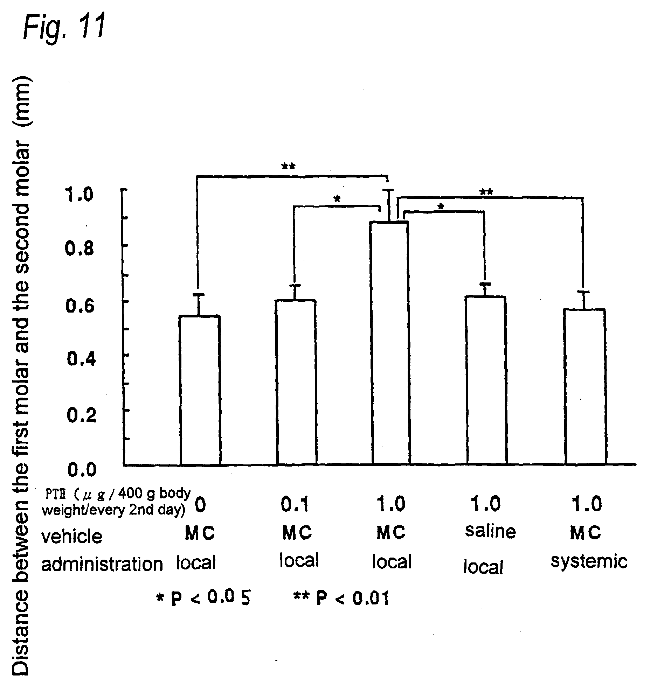

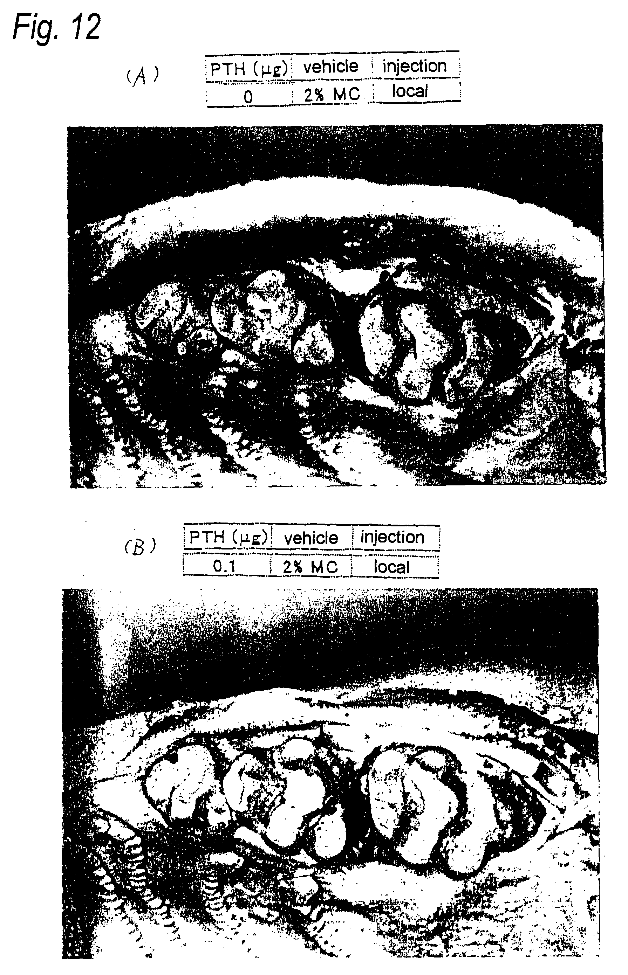

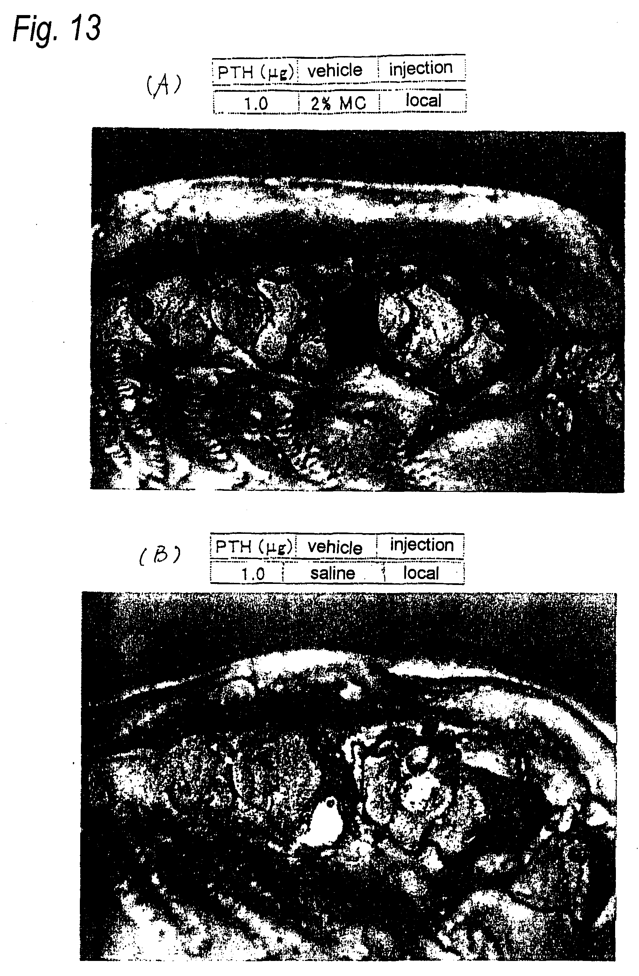

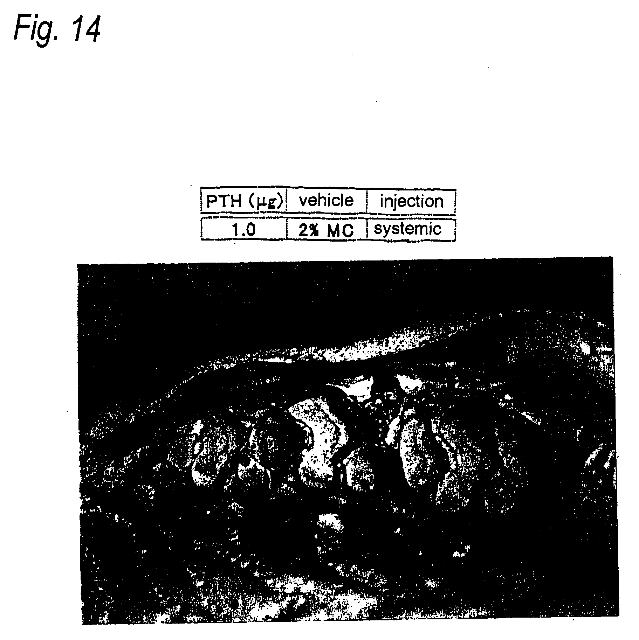

### 3. Effect of local injection of sustained release PTH (1-34) on experimental tooth movement (Figs. 11, 12, 13 and 14).

On day 12 of tooth movement, the control group to which 2% methylcellulose alone had been given showed a mesial shift of M₁ of 0.54 ± 0.08 mm. In the groups with the local administration of PTH-MC, on the other hand, the shift of M₁ was promoted depending on the concentration. Namely, the group to which 1 µg/400 g/day of PTH had been administered every 2 days showed a mesial shift of M₁ of 0.08 ± 0.11 mm, i.e., almost 1.6 times as much as that in the control group. The group with the local administration of the PTH-physiological saline and the group with the subcutaneous administration of PTH-MC to the dorsal part each showed no promotion in tooth movement.

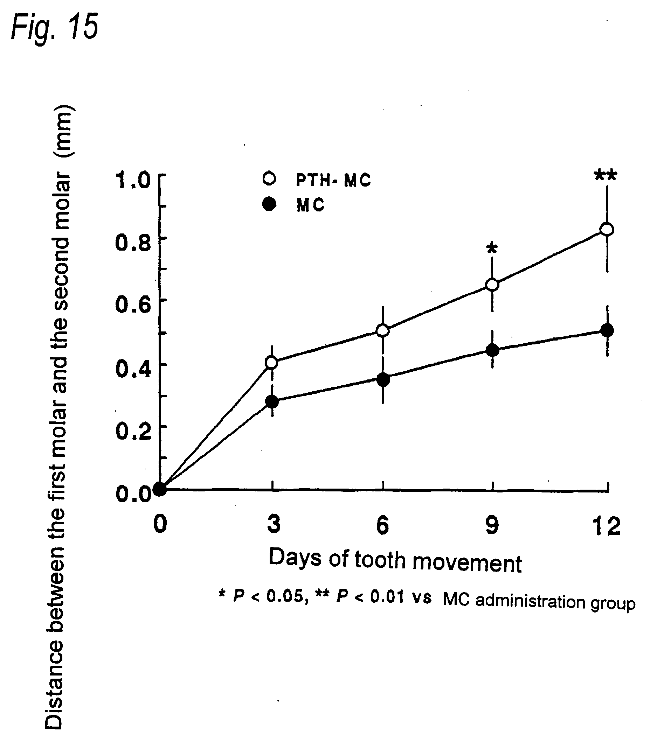

### 4. Change with the passage of time in effect of topical injection of sustained release PTH (1-34) on experimental tooth movement (Fig. 15).

When 1 µg/400 g of PTH (1-34) was locally injected every 2 days, there was observed a tendency that the mesial M₁ shift was promoted since day 3 of the tooth movement. This tooth movement-promoting effect of PTH became more remarkable after day 9.

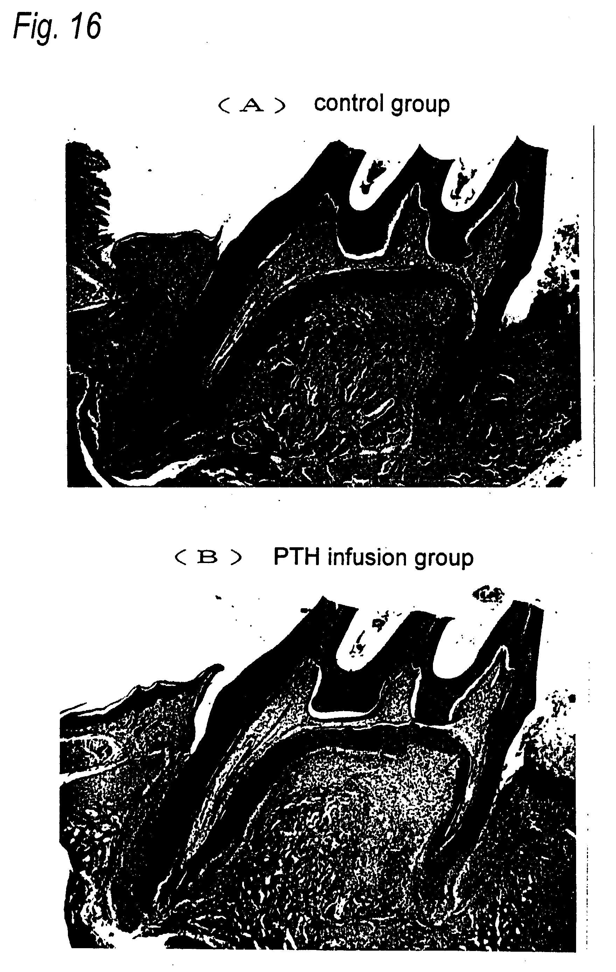

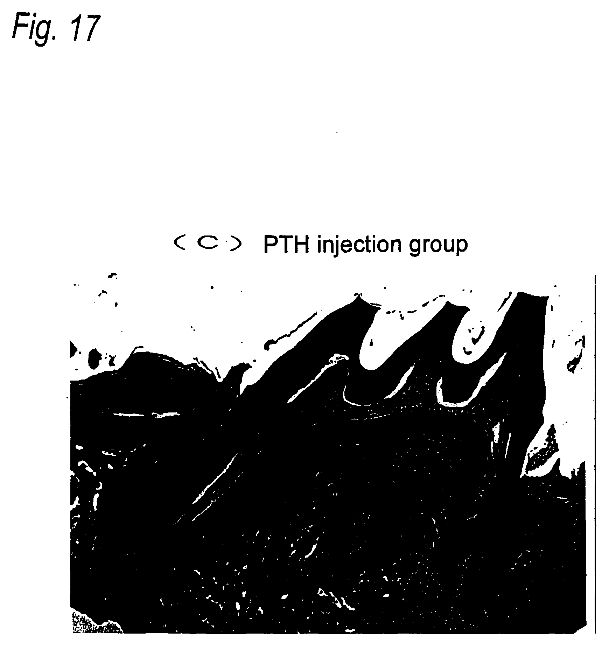

### 5. Histological findings in effects of the continuous PTH (1-34) infusion and the local injection of sustained release PTH on orthodontic tooth movement (Figs. 16, 17 and 18)

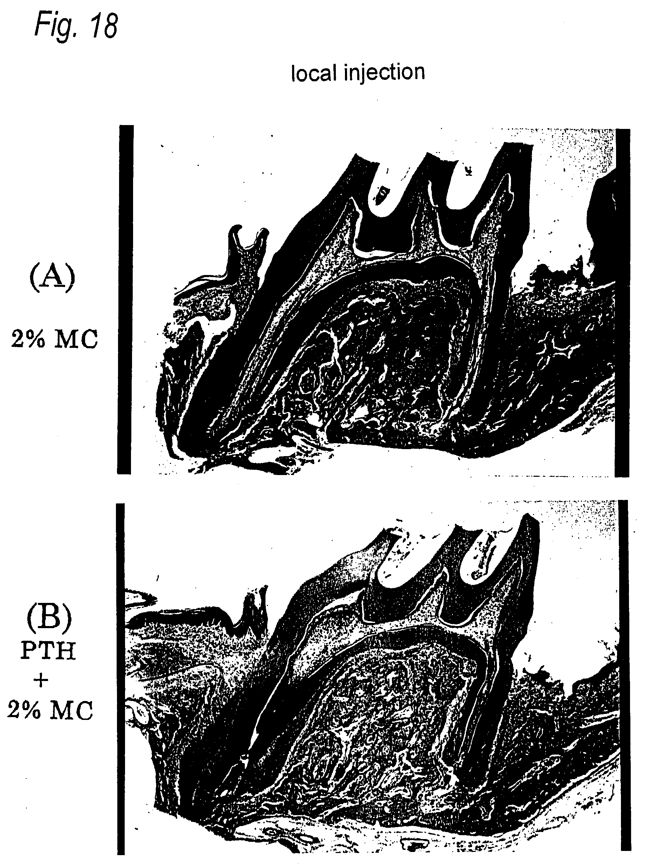

In the control group, a vitrified denaturation was observed in the periodontal membrane part between the alveolar septum and the distal root pressed by the traction force (Fig. 16-A). In the continuous infusion group, in contrast thereto, remarkable bone resorption was observed over a wide range in the distal side of the alveolar septum and no such necrotic tissue as observed in the control group was found (Fig. 16-B). Compared with the control group, the continuous infusion group further showed energetic bone resorption in the mesial alveolar bone of M₁. On the other hand, the group with the local injection of sustained release PTH showed no such alveolar bone resorption over a side range in compressed distal root side as observed in the group with the continuous systemic infusion (Fig. 18-B).

As described above, it has been proved that experimental tooth movement can be promoted by continuously administering PTH systemically or by locally injecting a PTH preparation in a sustained release dosage form.

### INDUSTRIAL APPLICABILITY

As described above, parathyroid hormone (PTH) or PTH derivatives have accelerated orthodontic tooth movement, which makes them useful as orthodontic remedies.

## Claims

1. An orthodontic remedy which contains parathyroid hormone (PTH) or one or more PTH derivatives as the active ingredient(s).

2. The orthodontic remedy as claimed in Claim 1 characterized in that said parathyroid hormone (PTH) is human PTH (1-84).

3. The orthodontic remedy as claimed in Claim 2 which contains substantially pure human PTH (1-84).

4. The orthodontic remedy as claimed in Claim 2 or 3 wherein said human PTH (1-84) is a recombinant human PTH (1-84).

5. The orthodontic remedy as claimed in Claim 1 characterized in that said PTH derivative is human PTH (1-34).

6. The orthodontic remedy as claimed in Claim 5 which contains substantially pure human PTH (1-34).

7. The orthodontic remedy as claimed in Claim 5 or 6 wherein said human PTH (1-34) is a recombinant human PTH (1-34).

8. The orthodontic remedy as claimed in Claim 1 characterized by containing parathyroid hormone (PTH) as the active ingredient.

9. The orthodontic remedy as claimed in Claim 8 characterized in that said parathyroid hormone (PTH) is human PTH (1-84).

10. The orthodontic remedy as claimed in Claim 9 which contains substantially pure human PTH (1-84).

11. The orthodontic remedy as claimed in Claim 9 or 10 wherein said human PTH (1-84) is a recombinant human PTH (1-84).

12. The orthodontic remedy as claimed in Claim 1 characterized by containing PTH derivative(s) as the active ingredient(s).

13. The orthodontic remedy as claimed in Claim 12 wherein said PTH derivative is human PTH (1-34).

14. The orthodontic remedy as claimed in Claim 13 which contains substantially pure human PTH (1-34).

15. The orthodontic remedy as claimed in Claim 13 or 14 wherein said human PTH (1-34) is a recombinant human PTH (1-34).

16. A dental composition which contains parathyroid hormone (PTH) or one or more PTH derivatives as the active ingredient(s).

17. The dental composition as claimed in Claim 16 characterized in that said parathyroid hormone (PTH) is human PTH (1-84).

18. The dental composition as claimed in Claim 16 characterized in that said parathyroid hormone (PTH) is human PTH (1-34).

19. A noninvasive PTH preparation characterized in that parathyroid hormone (PTH) or one or more PTH derivatives in an efficacious dose are continuously administered.

20. The noninvasive PTH preparation as claimed in Claim 19 wherein said noninvasive PTH preparation is the dental composition as claimed in Claim 16.
